# EUROPEAN PATENT APPLICATION

(11) **EP 2 270 137 A1**
(43) Date of publication of application: **05.01.2011**
(21) Application number: 09719288.4
(22) Date of filing: 11.03.2009
(51) Int. Cl.: C12N 5/10, A61K 35/12, A61P 37/04, C12N 15/09

(54) **METHOD FOR PRODUCING CELLS HAVING CHARACTERISTIC OF HEMATOPOIETIC STEM CELLS/PROGENITOR CELLS**

(30) Priority: 11.03.2008 JP 2008061542
(71) Applicant: Riken, Wako-shi, Saitama 351-0198 (JP); Katsura, Yoshimoto, Chiba 270-1166 (JP)
(72) Inventor: KATSURA, Yoshimoto, Abiko-shi Chiba 270-1166 (JP); KAWAMOTO, Hiroshi, Yokohama-shi Kanagawa 230-0045 (JP); IKAWA, Tomokatsu, Yokohama-shi Kanagawa 230-0045 (JP)
(74) Representative: Vossius & Partner
(86) International application number: PCT/JP2009/054698
(87) International publication number: WO 2009/113595

(57) **Abstract**

Provided are a new method of producing cells having characteristics of hematopoietic stem/progenitor cells, for use in hematopoietic stem cell transplantation, and hematopoietic stem/progenitor cell-like cells produced by the method. Provided in particular are a method of producing hematopoietic stem/progenitor cell-like cells retaining differentiation pluripotency and self-replication potential, comprising (1) a step for providing a mammalian pro-B cell or progenitor cell thereof, and (2) a step for culturing the cell (1) above under conditions for induction of differentiation into B cells, wherein the function and/or expression of the transcription factor E2A is suppressed at least at the stage of pre-pro-B cells or pro-B cells in the process (2) above; a hematopoietic stem/progenitor cell-like cell produced by the method; and an immunotherapeutic agent comprising the hematopoietic stem/progenitor cell-like cell, and the like.

## Description

### Technical Field

The present invention relates to a method of producing cells having characteristics of hematopoietic stem cells or hematopoietic progenitor cells (hereinafter, abbreviated as "hematopoietic stem/progenitor cells"), to be used as a source of progenitor cells in hematopoietic stem cell transplantation and cytotherapy using blood cells, a hematopoietic stem/progenitor cell-like cell produced by the method, an immunotherapeutic agent comprising as an active ingredient the cell or a cell differentiated from the cell and the like.

### Background Art

Bone marrow transplantation is performed as a trump in the treatment of tumoral diseases of the blood system and diseases due to abnormalities of hematopoietic cells, such as fatal anemia and immune deficiency. However, it is difficult to secure a donor because the donor's and patient's HLA types must be identical, and also because a considerable number of cells are required for bone marrow transplantation so that the donor unavoidably suffers burdens such as general anesthesia. Recently, hematopoietic stem cell transplantation using umbilical blood has also been spreading, but it does not significantly differ from bone marrow transplantation in the issue of HLA type matching, and a sufficient amount to enable use for adult treatment is not always obtainable, so that there is a limitation on the application to adults. Furthermore, in the event that cells transplanted from another person do not take, it is extremely difficult to perform transplantation again.

Hematopoietic stem cells are known to be maintained by self-replication in the bone marrow throughout the lifespan. Hence, in the stationary state, they are only maintained without major increases or decreases in total, and are not always multiplying. It is known, however, that when a few hematopoietic stem cells are transplanted by hematopoietic stem cell transplantation and the like, they multiply considerably in vivo. In mice, for example, it has been reported that if bone marrow transplantation is performed under certain conditions, hematopoietic stem cells multiply several tens of folds. This finding shows that hematopoietic stem cells are essentially multipliable. Hence, to secure a sufficient number of hematopoietic stem cells for use in hematopoietic stem cell transplantation, many attempts have been made to multiply hematopoietic stem cells ex vivo (Non-patent Document 1). These can be divided into roughly two types: attempts to reproduce an in vivo environment for hematopoietic stem cells ex vivo and attempts to reproduce a state that occurs in hematopoietic stem cells during self-replication.

Available methods of reproducing an in vivo environment include methods using external factors such as growth factors (Patent Documents 1 to 9, Non-patent Document 2). For example, there is a method wherein mainly growth factors such as cytokines are added to the medium so as to prevent the hematopoietic stem cells from dying outside of the body, or from losing the potential for stem cells. SCF, IL6, TPO, GCSF and the like are effective over a given period. To date, no cytokines capable of allowing stem cells to multiply efficiently by themselves alone have been found; although the effect increases when a plurality of cytokines are combined, this is not at a level allowing infinite multiplication of hematopoietic stem cells. As external factors, other than cytokines, that are involved in the maintenance and multiplication of hematopoietic stem cells, Notch, Wnt, Kirre and the like are known; however, all attempts using these environmental factors have failed to obtain an effect that will lead to an application (Non-patent Documents 3 and 4).

As methods of reproducing a state that occurs in hematopoietic stem cells, a method wherein an activation state of an intracellular signal received via a cytokine receptor is reproduced, a method wherein a transcription factor involved in the maintenance of the undifferentiation of stem cells or the like is transferred, and the like have been investigated (Patent Documents 10 to 12). For example, by transferring the Stat molecule, it is possible to multiply stem cells ex vivo (Patent Document 12). In the forcible expression of HoxB4, ex vivo multiplication to some extent has been successful (Non-patent Document 5). Bmi-1, one of polycomb molecules, which are suppression control factors, the suppression factor lnk for c-kit molecule downstream signals and the like are also known to be involved in the maintenance of stem cells, and have been investigated for applications to ex vivo multiplication of stem cells (Non-patent Documents 6 and 7) . Even in these cases, however, it is not possible to multiply hematopoietic stem cells in large amounts.

As stated above, many studies have been conducted energetically, but any attempts to multiply hematopoietic stem cells in large amounts ex vivo using a strategy based on the idea of reproducing physiological conditions have not yet been successful. Because the number of hematopoietic stem cells is kept at a constant level in vivo throughout the lifespan, and there is no particular multiplication except in special cases, it is likely to be impossible to achieve multiplication in large amounts ex vivo, as far as physiological conditions are utilized, judging from the principle involved. Therefore, to realize mass multiplication of hematopoietic stem cells, it is necessary to make an approach distinct from the conventional approach.

It has been shown to date that the transcription factor E2A is a factor that determines the destination of cells to differentiate into the B cell series by inhibiting differentiation into cells other than B cells, and functions at the turning point of commitment from pre-pro-B cells to pro-B cells. It was recently reported that in E2A-deficient mice, B cells are not produced in the B cell differentiation process, with the differentiation ceasing at the pre-pro-B cell stage (Non-patent Document 8), and that this E2A-deficient pre-pro-B cell exhibits characteristics for pluripotent progenitor cells (Non-patent Document 9). In these studies, however, mouse E2A was genetically deleted, and such elimination of the gene is extremely difficult to achieve in patient blood cells. Additionally, in the absence of E2A, mice are unable to produce B cells, that is, an antibody cannot be prepared; therefore, this pre-pro-B cell is useless as a progenitor cell for reconstruction of blood cells/immune cells. Therefore, it is impossible to use the same approach as this study to restore the immune potential of the patient.

The transcription factor PAX5, which is under the control of E2A, functions importantly in the process of differentiation into B cells; in PAX5-deficient mice, B cell differentiation ceases at the stage of pro-B cells. Additionally, there is the finding that this PAX5-deficient pro-B cell retains the potential for differentiation into T cells (Non-patent Document 10). As a related technology based thereon, Patent Document 13 discloses that cells retaining the potential for differentiation into T cells are obtained by inactivating the PAX5 gene in pro-B cells using a conditional knockout mouse just before use, and used to treat immunodeficiency of the lymphocyte system. However, there is no description concerning the multiplication of cells corresponding to hematopoietic stem/progenitor cells.
Therefore, as the situation stands, there is no method of optionally multiplying hematopoietic stem cells having characteristics of hematopoietic stem/progenitor cells for use in hematopoietic stem cell transplantation in large amounts ex vivo.
patent document 1: JP-A-2006-67858
patent document 2: JP-A-2006-61106
patent document 3: JP-A-2005-204539
patent document 4: JP-A-2004-222502
patent document 5: JP-A-2001-161350
patent document 6: JP-A-10-295369
patent document 7: National Publication of International
Patent Application No. 2006-525013
patent document 8: WO 2003/038077
patent document 9: WO 98/08869
patent document 10: JP-A-2007-37401
patent document 11: National Publication of International
Patent Application No. 2007-507206
patent document 12: National Publication of International
Patent Application No. 2006-505266
patent document 13: USP 20040029271
non-patent document 1: Nature Rev. Immunol. 4, 878-888, 2004
non-patent document 2: Science 316, 590-593, 2007
non-patent document 3: Nature 423, 409-414, 2003
non-patent document 4: Nature Immunol. 4, 457-463, 2003
non-patent document 5: Cell 109, 39-45, 2002
non-patent document 6: Nature 423, 255-260, 2003
non-patent document 7: Dev. Cell 8, 907-914, 2005
non-patent document 8: Immunity 6, 145-154, 1997
non-patent document 9: Immunity 20, 349-360, 2004
non-patent document 10: Nature 401, 556-562, 1999

### Disclosure of the Invention

### Problems to Be Solved by the Invention

It is an object of the present invention to provide a new method that enables mass multiplication of cells having characteristics of hematopoietic stem/progenitor cells ex vivo. Means of Solving the Problems

The present inventors conducted investigations to accomplish the above-described objective, and found that by transferring Id3, a differentiation suppression factor that inhibits E2A function, to mouse hematopoietic progenitor cells using a retrovirus vector to forcibly express Id3, differentiation is terminated at the stage of pre-pro-B cells as in cases where the E2A gene is knocked out, and that pre-pro-B cells can be mass-multiplied when cultivation under B cell culturing conditions is continued. Moreover, when transplanted to mice, these cells exhibited pluripotency and were able to maintain the hematopoietic potential for many series, including B cells, for a long period. Furthermore, by transplanting the cell to mice exposed to a lethal dose of radiation, the mice were successfully salvaged from exposure death. Based on these findings, the present inventors concluded that the cell is a cell having characteristics of hematopoietic stem/progenitor cells, and retaining differentiation pluripotency and self-replication potential.
The present inventors also succeeded in mass-multiplying CD33-positive CD19-negative cells by transferring the Id3 gene to human hematopoietic progenitor cells in the same way, and culturing the cells under B cell culturing conditions.
Furthermore, the present inventors succeeded in inducing cells having characteristics of hematopoietic stem/progenitor cells using a PI polyamide, which is a non-nucleic acid compound capable of binding specifically to the target DNA of E2A protein, in place of transferring the Id3 gene, and have developed the present invention.

Accordingly, the present invention relates to the following:
[1] A method of producing hematopoietic stem/progenitor cell-like cells retaining differentiation pluripotency and self-replication potential, comprising the following steps:
   (1) a step for providing a mammalian pro-B cell or progenitor cell thereof, and
   (2) a step for culturing the cell according to (1) above under conditions for induction of differentiation into B cells, wherein the function and/or expression of the transcription factor E2A is suppressed at least at the stage of pre-pro-B cells or pro-B cells in the step (2) above.
[2] The method according to [1] above, wherein the function of E2A is suppressed using an Id factor.
[3] The method according to [1] above, wherein the expression of E2A is suppressed using an antisense nucleic acid, siRNA or ribozyme against the E2A gene.
[4] The method according to [1] above, wherein the function of E2A is suppressed by suppressing the function and/or expression of another transcription factor that is under the control of E2A, or that works in cooperation with E2A.
[5] The method according to [1] above, wherein the function of E2A is suppressed using a pyrrole-imidazole polyamide.
[6] The method according to any one of [1] to [5] above, wherein the mammal is a human.
[7] The method according to any one of [1] to [6] above, wherein the pro-B cell progenitor cell is selected from the group consisting of hematopoietic stem cells, hematopoietic progenitor cells, lymphomyeloid series progenitor cells, pre-pro-B progenitor cells, ES cells and iPS cells.
[8] A hematopoietic stem/progenitor cell-like cell retaining differentiation pluripotency and self-replication potential, that can be produced by the method according to any one of [1] to [7] above.
[9] A cellular immunotherapeutic agent comprising the cell according to [8] above.
[10] A method of producing a blood cell, comprising culturing the cell according to [8] above under conditions for induction of differentiation into blood cells.
[11] A mature blood cell that can be produced by the method according to [10] above.
[12] A cellular immunotherapeutic agent comprising the cell according to [11] above, or a cell population in the midst of differentiation into the mature blood cell.
[13] The agent according to [9] or [12] above, further comprising a marrow cell.

### Effect of the Invention

Using the method of the present invention, with the only provision that HLA types matches each other in bone marrow transplantation, it is possible to prepare a cell having characteristics of hematopoietic stem/progenitor cells on the basis of a few cells, and multiply it nearly infinitely, so that the burden on the marrow fluid donor lessens significantly. Furthermore, because it also becomes possible to repeatedly perform transplantation using the multiplied cells, and to transplant the recipient's own cells as hematopoietic stem cells, the scope of application of transplantation widens, and even in cases where transplantation is currently unfeasible or unsuccessful because of limitations on the number of hematopoietic stem cells, transplantation can become feasible. Because various series of mature blood cells can be artificially created from a cell having characteristics of hematopoietic stem/progenitor cells, obtained by the method of the present invention, it is also possible to use the thus-obtained mature blood cells as cells for use in cytotherapy.

### Brief Description of the Drawings

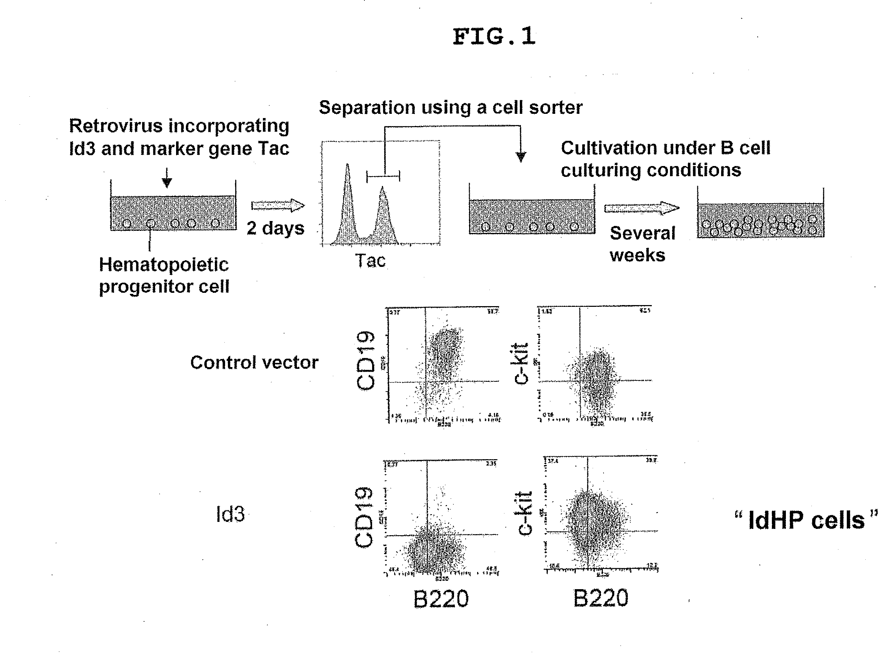

[Fig. 1] Fig. 1 shows a method of preparing mouse IdHP cells.

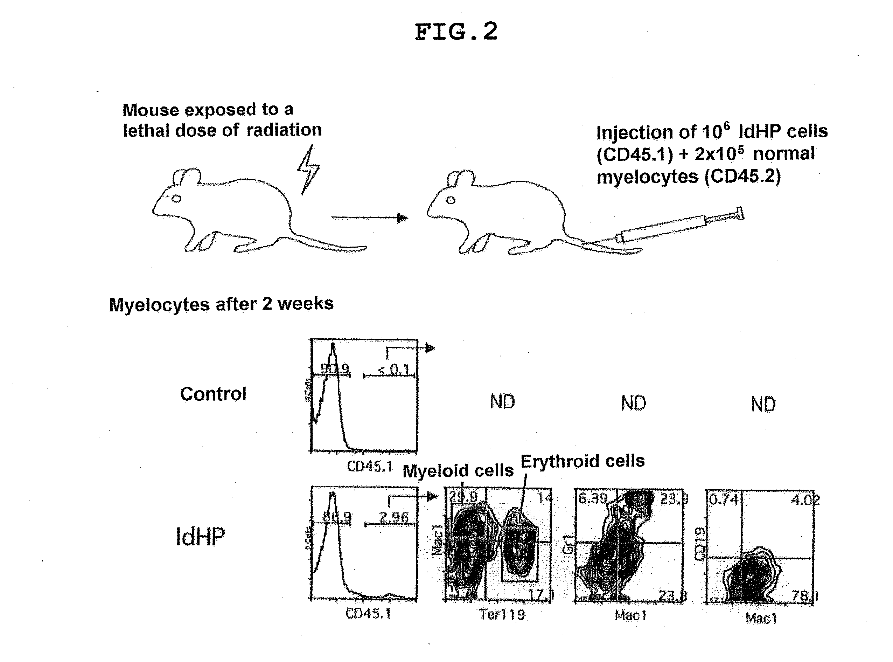

[Fig. 2] Fig. 2 shows the capability of hematopoietic reconstruction of mouse IdHP cells. By surface antigen marker analysis of IdHP cell-derived (CD45.1-positive) cells in marrow cells of mice as of 2 weeks after cell transfer, production of myeloid series and erythroid series cells was noted.

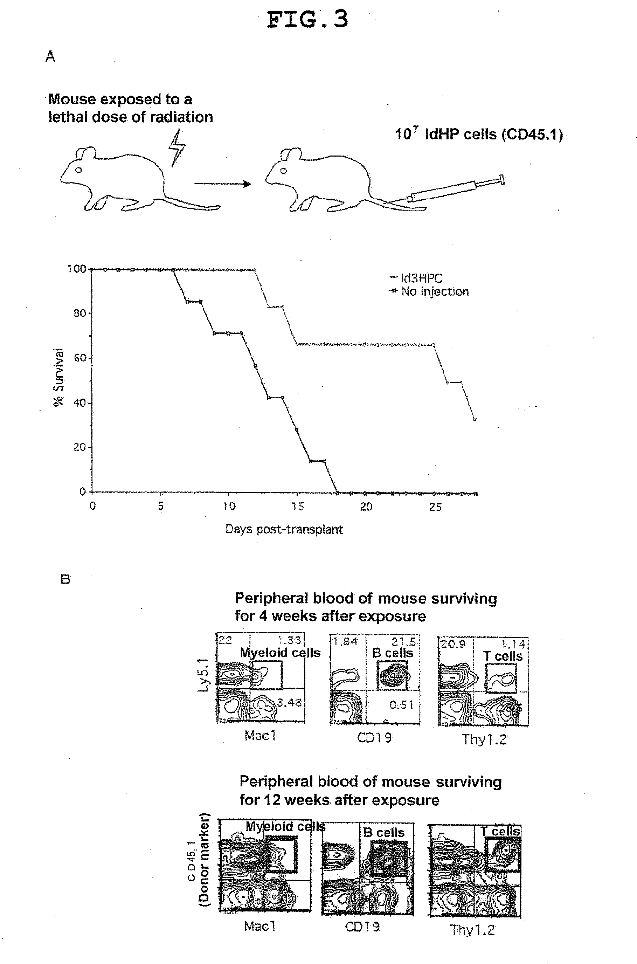

[Fig. 3] Fig. 3 shows the salvage of mice from exposure death using B progenitor cells having Id3 expressed forcibly therein. Fig. 3A shows a survival curve for mice having IdHP cells transferred thereto after being exposed to a lethal dose of radiation. Fig. 3B shows that blood cells derived from IdHP cells emerged in the peripheral blood of mice surviving for 4 weeks and 12 weeks after exposure.

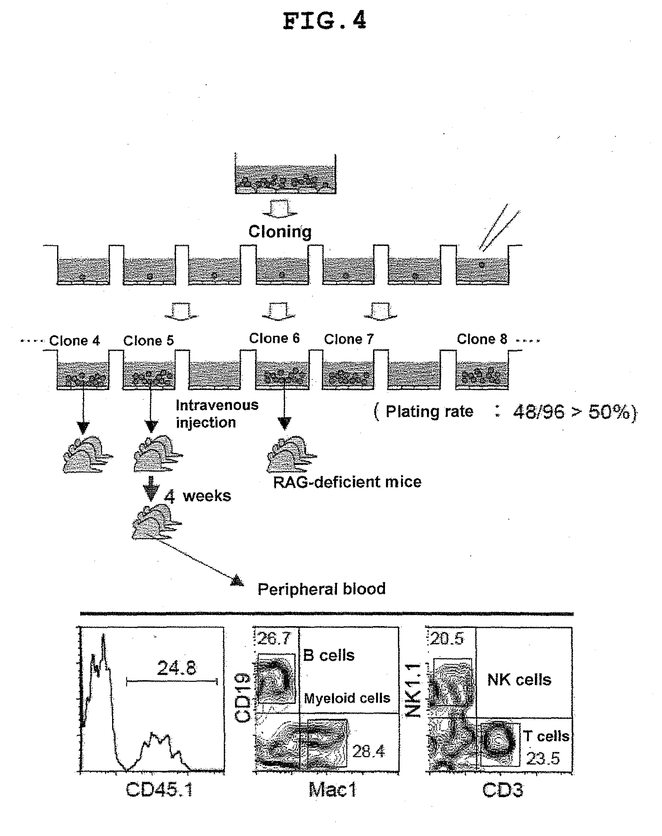

[Fig. 4] Fig. 4 shows that all of individual mouse IdHP cells are pluripotent progenitor cells. Each IdHP cell clone multiplied from a single mouse IdHP cell was intravenously injected; by surface antigen marker analysis of IdHP cell-derived (CD45.1-positive) cells in the peripheral blood of mice after 4 weeks, reconstruction of myeloid cells, B cells, T cells, and NK cells was confirmed.

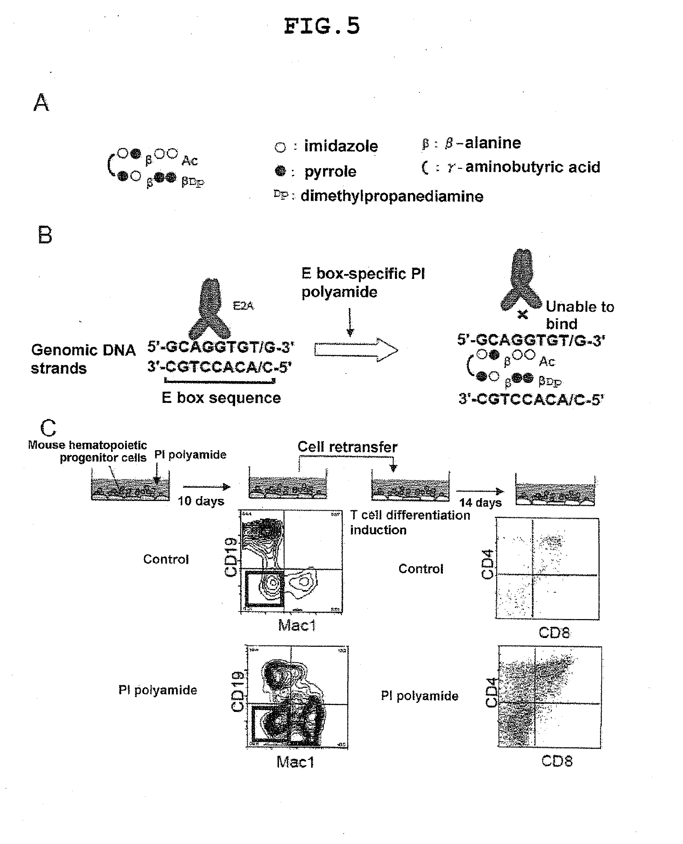

[Fig. 5] Fig. 5 shows that when the transcription regulatory function of E2A is inhibited with a synthetic PI polyamide that binds specifically to the E box sequence, which is a DNA binding motif of E2A (Fig. 5A) (Fig. 5B), hematopoietic progenitor cells having the potential for differentiation into T cells are multiplied (Fig. 5C).

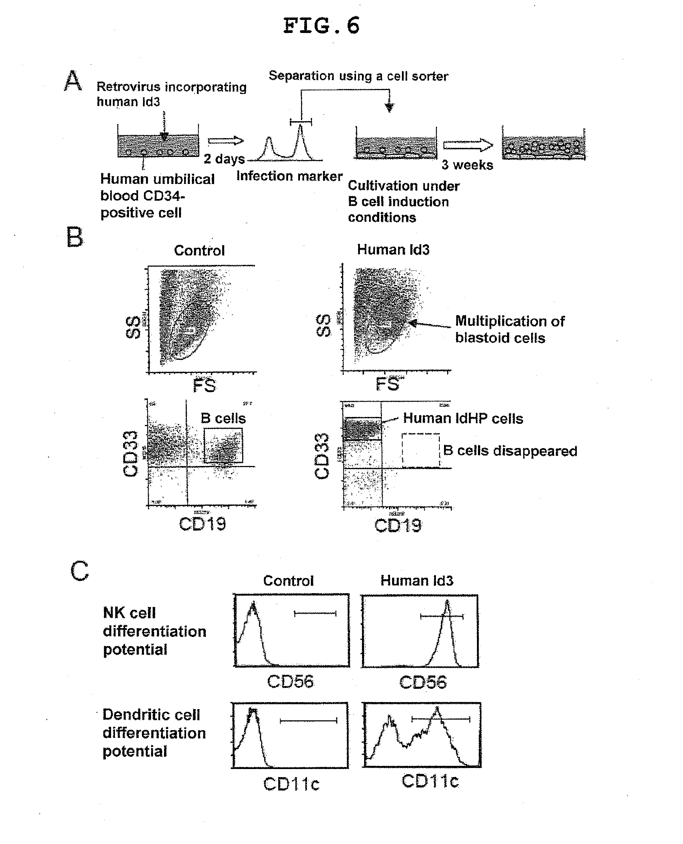

[Fig. 6] Fig. 6 shows the procedures for preparing human IdHP cells (Fig. 6A), the emergence of human IdHP cells after 4 weeks of cultivation (Fig. 6B), and that the human IdHP cells obtained possess pluripotency (Fig. 6C).

### Best Mode for Carrying out the Invention

The present invention provides a method of producing cells having characteristics of hematopoietic stem/progenitor cells. The method comprises:
(1) a step for providing a mammalian pro-B cell or progenitor cell thereof, and
(2) a step for culturing the cell according to (1) above under conditions for induction of differentiation into B cells, wherein the function and/or expression of the transcription factor E2A is suppressed at least at the stage of pre-pro-B cells or pro-B cells in the step (2) above.

Herein, "having characteristics of hematopoietic stem/progenitor cells" means retaining differentiation pluripotency and self-replication potential. "Differentiation pluripotency" means the potential for differentiation into a plurality of series of mature blood cells such as B cells, T cells, erythrocytes, and macrophages. "Self-replication potential" means the potential of a cell for continuing to multiply while retaining its characteristics.
Multiplication of hematopoietic stem/progenitor cells (retention of self-replication potential) can be evaluated by a cell marker analysis (for example, counting cells corresponding to various CD markers and the like using a cell sorter), a quantitative analysis based on the colony assay method and the like. The colony assay method is a method wherein hematopoietic cells are cultured in a semi-solid medium such as methyl cellulose or soft agar, and estimating the number and properties of hematopoietic stem cells and the like from the population of cells formed, that is, the colony. Retention of differentiation pluripotency can be measured by, for example, determining whether or not a hematopoietic stem cell has the potential for continuously producing a plurality of series of cells for a long period after being transferred to an adult mouse according to a method known per se (e.g., J Exp Med 192 (2000), 1281-1288). Alternatively, the same can also be achieved by culturing the subject cell under culturing conditions for induction of differentiation into various blood cells, and examining differentiation into the blood cells by cell marker analysis and the like.

Mammals to which the method of the present invention is applicable are not particularly limited, and can be, for example, humans, mice, rats, hamsters, monkeys, cattle, horses, pigs, sheep, goat, dogs, cats, guinea pigs, rabbits and the like, and are preferably humans.
The method of the present invention is intended to artificially prepare and mass-multiply hematopoietic stem/progenitor cell-like cells for cellular immunotherapy for mammals. Therefore, the mammal used in the method of the present invention is preferably the individual animal that is the subject of the cellular immunotherapy (autotransplantation) or an individual of the same species as the animal (allotransplantation), but this does not rule out the use of other animal species.

Herein, "a pro-B cell" is a B lymphocyte progenitor cell, characterized by the expression of surface antigen markers such as CD34, CD45R, B220, AA4.1, IL-7R, MHC class II, CD10, CD19, and CD38. "A pre-pro-B cell" is a cell that has differentiated from a lymphomyeloid series progenitor cell in differentiation into B lymphocytes, and that is at the stage before the pro-B cell stage (characterized by B220-positiveness and CD19-negativeness).
"The pro-B cell progenitor cell" is not particularly limited, as far as it is a cell that can be differentiated into pro-B cells; for example, in addition to hematopoietic stem cells, hematopoietic progenitor cells, lymphomyeloid series progenitor cells, and pre-pro-B progenitor cells, embryonic stem (ES) cells, induced pluripotent stem (iPS) cells and the like are also included in progenitor cells. Although ES cells are known to differentiate into various series of blood cells such as B cells, T cells, erythrocytes, and macrophages, there have been no successful cases of production of hematopoietic stem cells from ES cells. By suppressing the function and/or expression of E2A by the method of the present invention in ES/iPS cells or hematopoietic progenitor cells derived from ES/iPS cells, hematopoietic stem/progenitor cell-like cells can be produced.
The pro-B cells and progenitor cells thereof may be homogenous cells, and may be a heterogenous population of cells at various stages of differentiation.

Pro-B cells and progenitor cells thereof can be acquired from mammalian bone marrow, umbilical blood, peripheral blood and the like by a method known per se (for example, a method using an antibody against a surface antigen marker molecule and FACS (fluorescence activated cell sorter) and the like), or can be induced ex vivo. For example, because hematopoietic stem cells are characterized by CD34-negativeness or -weakly-positiveness, c-Kit-positiveness, Sca-1-positiveness, and differentiation antigen (Lineage marker)-negativeness (CD34^{-/low}KSL), they can be obtained by fractionating CD34^{-/low}KSL cells using antibodies against these surface antigens and FACS. The method of the present invention can be highly effective in case of, for example, autotransplantation in a patient with hematologic tumor (for example, marrow cells are collected before radiotherapy, and they are returned to the body after the treatment, and the like). Hence, in patients with hematologic tumor, the risk of the presence of tumor cells in the bone marrow is high; however, provided that progenitor cells that are distinguishable from tumor cells, for example, the above-described hematopoietic stem cells or pro-B cells, alone are separated with the expression of a surface antigen marker as an index using a cell sorter, tumor cells can be eliminated, so that autotransplantation can be performed safely.
Meanwhile, ES cells and iPS cells can be induced from early embryos and somatic cells by a method known per se.

Pro-B cells and progenitor cells thereof are cultured under conditions for induction of differentiation into B cells. Examples of media for inducing differentiation into B cells include a minimal essential medium (MEM) containing about 5 to 20% fetal calf serum (FCS), Dulbecco's modified Eagle medium (DMEM), Iscov's modified Dulbecco medium (IMDM), RPMI1640 medium, Ham's F12 medium and the like, supplemented with cytokines such as IL7, SCF, and Flt3 ligand. It is preferable that the cells be acclimated to a serum-free medium during the cultivation. The number of cells seeded to the medium is not particularly limited, as far as cultivation is possible, and the number is preferably about 1.0×10⁶ to about 1.0×10⁷ cells/ml. Duration of cultivation is not particularly limited, and is preferably 30 to 60 days. Culturing temperature is preferably about 30 to about 40°C, particularly preferably about 37°C. Carbon dioxide content is preferably about 5 to about 10%, particularly preferably about 5%.

The method of the present invention is characterized by suppressing the function and/or expression of the transcription factor E2A at least at the stage of pre-pro-B cells or pro-B cells in the above-described culturing step.
E2A is a transcription factor that determines the destination of a cell to differentiate into the B cell series by inhibiting differentiation into cells other than B cells, and reversibly functions at the turning point of commitment from pre-pro-B cells to pro-B cells. Therefore, if the function and/or expression of the factor is suppressed at the stage of pre-pro-B cells or pro-B cells, the differentiation into B cells ceases, and the cell reacquires the potential for differentiation into a plurality of cell series other than B cells (differentiation pluripotency). Because the cell does not proceed to differentiate into B cells, it is capable of self-replication while retaining differentiation pluripotency, and can be multiplied without limitations by continuing to be cultured under conditions for induction of differentiation into B cells.

In the present invention, "to suppress the function of E2A" may be in any mode, as far as it suppresses the once-functionally-produced E2A protein from controlling downstream gene expression to exhibit the activity to induce the differentiation of pre-pro-B cells into B cells; for example, enhancement of a factor that binds to E2A to suppress the acting of E2A on the target gene, transfer of a dominant negative mutant having the functional domain of E2A inactivated therein, or inhibition of the expression or activity of another transcription factor that is under the control of E2A, or that works in cooperation with E2A, and other methods can be mentioned.

Factors that bind to E2A to suppress the acting of E2A on the target gene include Id (Inhibitor of DNA binding) factors. Id factors are differentiation suppression factors occurring in 4 types (Id1 to Id4) in mammals, that inhibit the function of basic helix-loop-helix (bHLH) type transcription factors, which play an important role in cell differentiation. Id factors bind directly to E proteins, including E2A (E2A, HEB, E2-2), to suppress the functions thereof. The Id factors used in the present invention are not particularly limited; all of Id1 to Id4 are preferably usable.

Other factors that bind to E2A to suppress the acting of E2A on the target gene include, for example, antibodies against E2A and decoy nucleic acids having an E2A binding motif (e.g., 5'-AACAGATGGT-3'; SEQ ID NO:1, 5'-GCAGGTG(T/G)-3'; SEQ ID NO:2) and the like.

As a method of enhancing a factor that binds to E2A to suppress the acting of E2A on the target gene, the factor itself may be transferred into cells (for example, addition of the factor to the medium for pro-B cells or progenitor cells thereof, or transfer of the factor enclosed in a liposome into the cell and the like); however, to ensure that the factor is transferred into cells efficiently and supplied persistently, it is more preferable that an expression vector comprising a nucleic acid that encodes the factor be transferred to the cell and expressed forcibly.

For example, when Id3 is used as a factor that binds to E2A to suppress the acting of E2A on the target gene, an appropriate primer is designed on the basis of base sequence information on the Id3 gene (registered with GenBank under Refseq No. NM_002167 (human) and NM_008321 (mouse)), RT-PCR is performed with an RNA extracted from an Id3-expressing cell/tissue by a method known per se as a template, and a DNA comprising the base sequence that encodes Id3 can be multiplied and cloned. Alternatively, a cDNA of Id3 can be cloned from a cDNA library derived from the foregoing cell/tissue, using the hybridization method. For other Id factors, cloning can be achieved in the same way. The hybridization can be performed according to, for example, the method described in Molecular Cloning, 2nd edition (J. Sambrook et al., Cold Spring Harbor Lab. Press, 1989) and the like.
The cloned DNA can be used as is, or after digestion with a restriction endonuclease or addition of a linker as desired, depending on the purpose of its use, as joined downstream of a promoter matching the host mammalian pro-B cell or progenitor cell thereof. The DNA may have the translation initiation codon ATG at the 5' end thereof, and the translation stop codon TAA, TGA or TAG at the 3' end thereof. These translation initiation codons and translation stop codons can be added using an appropriate synthetic DNA adapter.

As the expression vector, animal cell expression plasmids (e.g., pA1-11, pXT1, pRc/CMV, pRc/RSV, pcDNAI/Neo); bacteriophages such as λ phage; animal virus vectors such as retrovirus, adenovirus, adeno-associated virus, and lentivirus, and the like are used. The promoter may be any promoter that well matches the host used for gene expression. For example, the SRα promoter, SV40 promoter, LTR promoter, CMV (cytomegalovirus) promoter, RSV (Rous sarcoma virus) promoter, MoMuLV (Moloney mouse leukemia virus) LTR, HSV-TK (herpes simplex virus thymidine kinase) promoter and the like are used.
Useful expression vectors include, in addition to the above, those optionally harboring an enhancer, a splicing signal, a polyA addition signal, a selection marker, an SV40 replication origin and the like. Selection markers include various drug resistance genes, genes that encode surface antigens that are not expressed by the host pro-B cell or progenitor cell thereof and the like.

Transfer of the gene to cells can be achieved by a method utilizing a virus such as retrovirus, adenovirus, adeno-associated virus, lentivirus, or Sendai virus, and a method wherein a simple plasmid vector is transferred into cells using electroporation, liposome fusion, a gene gun and the like, and the like.
Because permanent expression is thought to be essential to maintain the self-replication potential as stem cells in vivo, integration of the gene in the chromosome is preferred. Because a lentivirus is stably integrated in chromosomal DNA and seldom undergoes epigenetic silencing, it is preferable for the purpose of permanently maintaining the self-replication potential of hematopoietic stem/progenitor cell-like cells; however, there is the drawback that permanent suppression of the function of E2A makes the cell unable to reacquire the potential for differentiation into B cells. Meanwhile, if a retrovirus is used, the gene integrated in the chromosome becomes inactivated at a certain ratio and is no longer expressed, so that the function of E2A is restored at a certain ratio, and the population of cells treated to transfer the gene, as a whole, will reacquire the potential for differentiation into B cells as well, while retaining the self-replication potential and the potential for differentiation into a plurality of cell series other than B cells. Therefore, in case of overexpression, a retrovirus is preferably used. Because a persistent expression type Sendai virus vector (e.g., J. Biol. Chem. 282, 27383-27391, 2007) is capable of being stably present outside of the chromosome, and can be degraded and removed using an siRNA if required, it is likewise used preferably.
When genetically modified cells are prepared, the risk of transformation to leukemia in vivo must be born in mind. In the method of the present invention, in mouse experiments, within the period of follow-up observation for up to 4 months, fatal leukemia has not been observed, so that the risk is not thought to be always high. However, to ensure safety, it is also possible to use in combination a method wherein a suicide gene is also transferred at the same time to allow the transferred cells to be eliminated by drug administration after the transferred cells exhibit a specified effect, in the event of transformation to leukemia, and the like. Suicide genes include thymidine kinase, ganciclovir as a cell death inducer and the like.
Meanwhile, in methods of utilization, for example, transplantation intended to recover the immune potential by transient hematopoiesis, and use for cytotherapy after ex vivo differentiation induction, rather than as stem cell transplantation, suppression of E2A by direct administration of a suppression factor is preferred to transfection of a suppression factor using a virus and the like, because the gene of the patient's cells need not to be modified.

Because the cells after gene transfer terminate their differentiation into B cells because of suppression of the function of E2A, the cells can be maintained and mass-multiplied for a long period while retaining the characteristics of hematopoietic stem/progenitor cells by continuing to culture them under the above-described pro-B cell culturing conditions.

Other transcription factors that are under the control of E2A, and that work in cooperation with E2A to act in the process of B cell differentiation include, for example, EBF, PAX5 and the like. Methods of suppressing the functions of these other transcription factors include, for example, transfer of a dominant negative mutant having a functional domain thereof inactivated therein and the like. Specifically, a DNA that encodes EBF or PAX5 is isolated by the same technique as the above-described case of Id3, a mutation is introduced to the functional domain of the DNA by a commonly known method of site-directed mutagenesis and the like to prepare a DNA that encodes a dominant negative mutant, and this DNA is transferred to the host pro-B cell or progenitor cell thereof by the same technique as the above and the like.

The above-described method of suppressing the expression of other transcription factors can be performed in the same way as the method of suppressing the expression of E2A, described below.

In the present invention, "to suppress the expression of E2A" may be suppression of E2A at any stage of the E2A gene, including the transcription level, posttranscriptional regulation level, translation-into-protein level, posttranslational modification level and the like. Therefore, substances that suppress the expression of E2A include, for example, a substance that inhibits the transcription of the gene, a substance that inhibits the processing of early transcription product to mRNA, a substance that inhibits the transportation of mRNA to cytoplasm, a substance that promotes the degradation of mRNA, a substance that inhibits the translation of mRNA to protein, a substance that inhibits the posttranslational modification of E2A polypeptide and the like. Although a substance that acts at any stage can be preferably used, a substance that inhibits the translation of mRNA to protein is preferred in the sense of directly inhibiting the production of E2A protein.

As a preferable substance capable of specifically inhibiting the translation of the mRNA of E2A to protein, a nucleic acid comprising a base sequence complementary to the base sequence of the mRNA or a portion thereof can be mentioned. More specifically, base sequences complementary to the base sequence of the mRNA of E2A include base sequences capable of hybridizing with the base sequences of the mRNA of E2A registered with GenBank under accession No. M31523 (human) and NM_011548.3 (mouse) under stringent conditions. As examples of stringent conditions, a hybridization reaction at 45°C in 6×SSC (sodium chloride/sodium citrate) followed by not less than one time of washing at 65°C in 0.2×SSC/0.1% SDS, and the like can be mentioned.

Although "a portion of a base sequence complementary to the base sequence of the mRNA of E2A" is not particularly limited with respect to the length and position thereof, as far as it is capable of binding specifically to the mRNA of E2A, and it is capable of inhibiting the translation of the mRNA to protein, it is preferable from the viewpoint of sequence specificity that the portion comprise at least 10 bases or more, preferably about 15 bases or more, more preferably about 20 bases or more, of a portion complementary or substantially complementary to the target sequence.

Specifically, the nucleic acid comprising a base sequence complementary or substantially complementary to the base sequence of the mRNA of E2A or a portion thereof is preferably one of the following (a) to (c).
(a) Antisense nucleic acid against mRNA of E2A
(b) siRNA against mRNA of E2A
(c) Ribozyme against mRNA of E2A

### (a) Antisense nucleic acid against mRNA of E2A

"An antisense nucleic acid against the mRNA of E2A" in the present invention is a nucleic acid that comprises a base sequence complementary to the base sequence of the mRNA or a portion thereof, and that exhibits the function to suppress protein synthesis by binding to the target mRNA to form a specific and stable double strand.

Examples of the antisense nucleic acid include polydeoxyribonucleotides comprising 2-deoxy-D-ribose, polyribonucleotides comprising D-ribose, other types of polynucleotides which are N-glycosides of the purine or pyrimidine base, other polymers having a non-nucleotide backbone (for example, commercially available nucleic acid polymers specific for protein nucleic acids and synthetic sequences) or other polymers comprising a special linkage (with the provision that the polymers comprise nucleotides having an alignment that allows base pairing or base attachment, as found in DNA or RNA) and the like. These may be double-stranded DNAs, single-stranded DNAs, double-stranded RNAs, single-stranded RNAs, or DNA:RNA hybrids, and may also be unmodified polynucleotides (or unmodified oligonucleotides); those with known modifications, for example, those with labels known in the art, those with caps, those methylated, those with substitution of one or more naturally occurring nucleotides with their analogue, those with intramolecular modifications of nucleotides, for example, those with uncharged linkages (for example, methyl phosphonates, phosphotriesters, phosphoramidates, carbamates and the like) and those with charged linkages or sulfur-containing linkages (e.g., phosphorothioates, phosphorodithioates and the like); those having side chain groups, for example, proteins (e.g., nucleases, nuclease inhibitors, toxins, antibodies, signal peptides, poly-L-lysine and the like) or saccharides (e.g., monosaccharides and the like); those with intercalators (e.g., acridine, psoralen and the like); those with chelators (for example, metals, radioactive metals, boron, oxidative metals and the like); those with alkylating agents; or those with modified linkages (for example, α anomeric nucleic acids and the like). Here, "nucleoside", "nucleotide" and "nucleic acid" may comprise not only the purine and pyrimidine bases, but also other modified heterocyclic bases. Such modified products may comprise a methylated purine and pyrimidine, an acylated purine and pyrimidine, or another heterocyclic ring. The modified nucleoside and modified nucleotide may have a modification in the sugar moiety thereof; for example, one or more hydroxyl groups may be substituted with halogens, aliphatic groups and the like, or may be converted into functional groups such as ethers and amines.

As stated above, the antisense nucleic acid may be a DNA or RNA, or a DNA/RNA chimera. When the antisense nucleic acid is a DNA, the RNA:DNA hybrid formed by the target RNA and antisense DNA is capable of being recognized by endogenous RNase H to cause selective degradation of the target RNA. Therefore, in case of an antisense DNA intended to cause degradation by RNase H, the target sequence may be not only a sequence in the mRNA, but also the sequence of an intron region in the early translation product of the E2A gene. For example, in humans, because the E2A gene is present in the 19p13.3 region of chromosome number 19, it is possible to determine the intron sequence by comparing the genome sequence of the region and the human E2A cDNA sequence registered with GenBank under accession No. M31523 using a homology search program such as BLAST or FASTA.

The target region for the antisense nucleic acid of the present invention is not particularly limited with respect to the length thereof, as far as hybridization of the antisense nucleic acid results in the inhibition of the translation into E2A protein; the target region may be the entire sequence or a partial sequence of the mRNA that encodes the protein, and may be a sequence of about 10 bases for the shortest, or of the entire sequence of the mRNA or initial transcription product for the longest. Taking into account the issues of the ease of synthesis and intracellular migration and the like, an oligonucleotide consisting of about 10 to about 40 bases, particularly about 15 to about 30 bases, is preferable, but this is not to be construed as limiting. Specifically, the 5' end hairpin loops, 5' end noncoding regions, translation initiation codons, protein coding regions, ORF translation stop codons, 3' end noncoding regions, 3' end palindrome regions, 3' end hairpin loops and the like of the E2A gene can be chosen as preferable target regions for the antisense nucleic acid, but these are not to be construed as limiting.

Furthermore, the antisense nucleic acid of the present invention may be one capable of not only hybridizing with the mRNA or initial transcription product of E2A to inhibit the translation into protein, but also binding to these genes that are double-stranded DNAs to form a triple strand (triplex) and inhibit the transcription to RNA (antigene).

Although the nucleotide molecules that constitute the antisense nucleic acid may be naturally occurring DNAs or RNAs, the molecules can contain various chemical modifications in order to increase the stability (chemical and/or to-enzyme) or specific activity (affinity for RNA). For example, to prevent degradation by hydrolylases such as nuclease, the phosphoric acid residue (phosphate) of each nucleotide that constitutes the antisense nucleic acid can be substituted with, for example, a chemically modified phosphoric acid residue such as phosphorothioate (PS), methylphosphonate, or phosphorodithionate. The hydroxyl group at the 2'-position of the sugar (ribose) of each nucleotide may be replaced with -OR (R represents, for example, CH₃(2'-O-Me), CH₂CH₂OCH₃(2'-O-MOE), CH₂CH₂NHC(NH)NH₂, CH₂CONHCH₃, CH₂CH₂CN or the like). Furthermore, a base moiety (pyrimidine, purine) may be chemically modified; for example, introduction of a methyl group or a cationic functional group into the 5-position of the pyrimidine base, substitution of the 2-position carbonyl group with thiocarbonyl and the like can be mentioned.

Regarding the conformation of the sugar moiety of RNA, two types are dominant: C2'-endo (S type) and C3'-endo (N type); in a single-stranded RNA, the sugar moiety occurs in an equilibrium of the two, but when a double strand is formed, the conformation is fixed at the N type. Therefore, BNA (LNA) (Imanishi, T. et al., Chem. Commun., 1653-9, 2002; Jepsen, J.S. et al., Oligonucleotides, 14, 130-46, 2004) and ENA (Morita, K. et al., Nucleosides Nucleotides Nucleic Acids, 22, 1619-21, 2003), which are RNA derivatives wherein the conformation of the sugar moiety is fixed at the N type by bridging the 2' oxygen and 4' carbon to confer strong bindability to the target RNA, can also be used preferably.

An antisense oligonucleotide of the present invention can be prepared by determining the target sequence for the mRNA or initial transcription product on the basis of the cDNA sequence or genomic DNA sequence of E2A, and synthesizing a sequence complementary thereto using a commercially available automated DNA/RNA synthesizer (Applied Biosystems, Beckman and the like). All antisense nucleic acids comprising the aforementioned various modifications can be chemically synthesized by techniques known per se.

### (b) siRNA against mRNA of E2A

Herein, a double-stranded RNA consisting of an oligo-RNA complementary to the mRNA of E2A and a strand complementary thereto, i.e., what is called an siRNA, is also defined as being included in nucleic acids comprising a base sequence complementary to the base sequence of the mRNA of E2A or a portion thereof. It had been known that what is called RNA interference (RNAi), the phenomenon where transfer of a short double-stranded RNA into a cell results in the degradation of mRNAs complementary to the RNA, occurs in nematodes, insects, plants and the like; since this phenomenon was confirmed to occur widely in animal cells as well (Nature, 411(6836), 494-498 (2001), siRNA has been widely utilized as an alternative technique to ribozymes. An siRNA can be designed as appropriate on the basis of base sequence information on the mRNA serving as the target, using commercially available software (e.g., RNAi Designer; Invitrogen and the like).

The ribonucleoside molecules constituting the siRNA may also have the same modifications as the above-described case of antisense nucleic acid to improve the stability, specific activity and the like. However, in case of an siRNA, it is necessary to introduce the minimally modified nucleoside allowing the RISC complex to function because naturally occurring RNA can lose its RNAi activity if all ribonucleoside molecules therein are replaced with modified forms.

An siRNA can be prepared by synthesizing a sense chain and antisense chain of the target sequence on the mRNA using an automated DNA/RNA synthesizer, respectively, and denaturing the chains in an appropriate annealing buffer solution at about 90 to about 95°C for about 1 minute, and thereafter annealing the chains at about 30 to about 70°C for about 1 to about 8 hours. An siRNA can also be prepared by synthesizing a short hairpin RNA (shRNA) that serves as an siRNA precursor, and cleaving this using a dicer.

### (b') Nucleic acid capable of producing an siRNA against mRNA of E2A

Herein, a nucleic acid designed to produce the above-described siRNA against the mRNA of E2A in vivo is also defined as being included in nucleic acids comprising a base sequence complementary to the base sequence of the mRNA of E2A or a portion thereof. Such nucleic acids include the above-described shRNA and the like. An shRNA can be prepared by designing an oligo-RNA comprising a base sequence resulting from joining of a sense chain and antisense chain of the target sequence on the mRNA with a spacer sequence having a length enabling the formation of an appropriate loop structure (for example, from about 15 to 25 bases) inserted therebetween, and synthesizing this using an automated DNA/RNA synthesizer.

### (c) Ribozyme against mRNA of E2A

Other preferred examples of the nucleic acid comprising a base sequence complementary or substantially complementary to the base sequence of the mRNA of E2A or a portion thereof include ribozymes capable of specifically cleaving the mRNA in the coding region. In the narrow sense, "a ribozyme" refers to an RNA possessing an enzyme activity to cleave a nucleic acid, and is herein understood to be used as a concept encompassing DNA, as far as sequence-specific nucleic acid cleavage activity is possessed. The most versatile ribozymes are self-splicing RNAs found in infectious RNAs such as viroid and virusoid, and the hammerhead type, the hairpin type and the like are known. The hammerhead type exhibits enzyme activity with about 40 bases in length, and it is possible to specifically cleave the target mRNA alone by making several bases at both ends adjoining to the hammerhead structure portion (about 10 bases in total) to be a sequence complementary to the desired cleavage site of the mRNA. Because this type of ribozyme has RNA as the only substrate, it offers an additional advantage of non-attack to genomic DNA. Provided that the mRNA of E2A assumes a double-stranded structure by itself, the target sequence can be made single-stranded, using a hybrid ribozyme prepared by joining an RNA motif derived from a viral nucleic acid capable of binding specifically to RNA helicase (Proc. Natl. Acad. Sci. USA, 98(10) 5572-5577, 2001). Furthermore, when the ribozyme is used in the form of an expression vector containing the DNA that encodes it, the ribozyme may be a hybrid ribozyme prepared by further joining a sequence modified from the tRNA to promote the migration of the transcription product to cytoplasm (Nucleic Acids Res., 29(13) 2780-2788, 2001).

A nucleic acid comprising a base sequence complementary to the base sequence of the mRNA of E2A or a portion thereof can be supplied in a special form like a liposome or microspheres, and can be given in an adduct form. Such adduct forms used include polycations like polylysine, which act to neutralize the charge of the phosphate backbone, and hydrophobic compounds like lipids that enhance the interaction with the cell membrane or increase the uptake of nucleic acids (e.g., phospholipid, cholesterol and the like). As lipids preferred for addition, cholesterol and derivatives thereof (e.g., cholesterylchloroformate, cholic acid and the like) can be mentioned. These can be attached to the 3' end or the 5' end of nucleic acid, and can be attached via a base, a sugar or an intramolecular nucleoside bond. As other groups, a capping group arranged specifically at the 3' end or 5' end of nucleic acid to prevent degradation by nucleases such as exonuclease and RNase can be mentioned. Such capping groups include, but are not limited to, hydroxyl group protecting groups known in the relevant field, including glycols such as polyethylene glycol and tetraethylene glycol.

Regarding the nucleic acid comprising a base sequence complementary to the base sequence of the mRNA of E2A or a portion thereof, a DNA that encodes it can be inserted into an appropriate expression vector and transferred to a host pro-B cell or progenitor cell thereof in the same manner as the above-described case of a factor that binds to E2A to suppress the acting of E2A on the target gene. Regarding antisense nucleic acids and ribozymes, the same expression vectors, promoters and the like as those mentioned above can be utilized, respectively. As the shRNA expression vector, one having a Pol III system promoter such as U6 or H1 can be used. In this case, an shRNA transcribed in the cell incorporating the expression vector forms a loop by itself, and is thereafter processed by an endogenous enzyme dicer and the like, whereby a mature siRNA is formed.
Transfer of the gene to cells and cultivation of the cells after the transfer can also be performed in the same manner as the above.

As another technique for suppressing the function and expression of E2A, a method using a pyrrole-imidazole (PI) polyamide can be mentioned. Of PI polyamides, the pyrrole (Py)/imidazole (Im) pair recognizes CG, the Py/Py pair recognizes AT or TA, and the Im/Py pair recognizes GC, whereby they are able to bind to a wide variety of optionally chosen double-stranded DNAs base-specifically to suppress the expression of the target gene. Additionally, because a PI polyamide enters and binds to a minor group in DNA thereby to inhibit the binding of transcription factors to the DNA, it is possible to suppress the function of E2A by suppressing the expression of a gene under the control of E2A using a PI polyamide that binds to the E box sequence (5'-GCAGGTG(T/G)-3'; SEQ ID NO:2), which is an E2A binding cis-element of the gene (Fig. 5A) (Fig. 5B). Because PI polyamides, unlike existing antisense nucleic acids and siRNAs, do not have a nucleic acid structure, they are unlikely to undergo degradation by nucleases in vivo, and do not require a drug delivery system such as a vector or cationic lipid, electroporation and the like.
A PI polyamide can be synthesized with, for example, a Py and Im derivative having an Fmoc protecting amino group (the formula below) as a starting material for synthesis, using Fmoc peptide solid phase synthetic technology, but this is not to be construed as limiting.

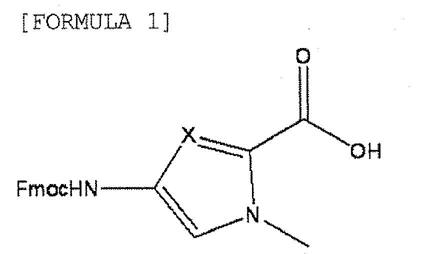

### (In the formula, X represents a carbon or nitrogen atom.)

In order to allow the PI polyamide to form a desired hairpin structure, an appropriate linker, for example, γ-aminobutyric acid, is introduced into the molecule between the pair of pyrrole and imidazole chains. An appropriate spacer molecule that does not contribute to the binding to the target DNA sequence, such as β-alanine, can be inserted into a position where the pair of pyrrole and imidazole chains are complementary to each other.

After completion of the coupling of the entire sequence of the PI polyamide, it is possible to cut out the PI polyamide from the solid phase by capping the terminal amino group with an acyl group or the like, and thereafter converting the polyamide to a carboxylic acid using trifluoroacetic acid (TFA), or to an amine using N,N-dimethylaminopropylamine (Dp).

The PI polyamide obtained can be isolated/purified by a commonly known method of purification. Here, methods of purification include, for example, solvent extraction, distillation, column chromatography, liquid chromatography, recrystallization, a combination thereof and the like. The purified PI polyamide can be stored at room temperature after being freeze-dried by a method known per se, and just before use, it can be dissolved in an organic solvent such as DMSO, and then diluted with water or a water/organic solvent mixture to an appropriate concentration.

As stated above, the PI polyamide is easily transferred to cells merely by being added to the medium without using a special means of transfer. The concentration of PI polyamide added is not particularly limited, as far as it is sufficient to suppress the function of E2A, and it is in a range where the growth of the cells is not adversely influenced; for example, the PI polyamide can be added to the medium to obtain concentrations of 5 to 50 µM.

The hematopoietic stem/progenitor cell-like cell thus obtained can be mass-multiplied for a long period while retaining self-replication potential and differentiation pluripotency by continuing to be subcultured under the above-described culturing conditions for induction of differentiation into B cells. Because the cell permits storage under freezing, it is possible to use part of the multiplied hematopoietic stem/progenitor cell-like cells for transplantation, while keeping the remainder stored under freezing, which is to be thawed just before use when retransplantation becomes necessary in the event of a failure of the graft to take and the like.

The present invention also provides a hematopoietic stem/progenitor cell-like cell retaining differentiation pluripotency and self-replication potential, that can be produced by the above-described method. In the hematopoietic stem/progenitor cell-like cell, the function or expression of E2A is suppressed permanently or transiently (or entirely or partially) depending on the means of suppression. Hematopoietic stem/progenitor cell-like cells undergoing suppression in different modes can exhibit respective excellent effects in the field of cellular immunotherapy. Therefore, the present invention also provides a cellular immunotherapeutic agent comprising the hematopoietic stem/progenitor cell-like cell.

Because the cellular immunotherapeutic agent of the present invention comprises cells having characteristics of hematopoietic stem/progenitor cells as an active ingredient, it can be used as a prophylactic and/or therapeutic agent for diseases accompanied by hematopoietic functional disorders, for example, aplastic anemia, congenital immunodeficiency, congenital metabolic disorders, myelodysplastic syndrome, leukemia, malignant lymphoma, multiple myeloma, myelofibrosis, radiation injuries and the like.

The cellular immunotherapeutic agent of the present invention can be used in an effective amount of the above-described hematopoietic stem/progenitor cell-like cell as it is, or after being blended with a pharmaceutically acceptable carrier to form a pharmaceutical composition according to a conventional means. The agent of the present invention is preferably produced as a non-oral preparation such as an injection, suspension, drip infusion and the like. Pharmaceutically acceptable carriers that can be contained in the non-oral preparation include, for example, aqueous solutions for injection, such as physiological saline and isotonic solutions containing glucose or another auxiliary drug (for example, D-sorbitol, D-mannitol, sodium chloride and the like). The agent of the present invention may be formulated with, for example, a buffering agent (for example, phosphate buffer solution, sodium acetate buffer solution), a soothing agent (for example, benzalkonium chloride, procaine hydrochloride and the like), a stabilizer (for example, human serum albumin, polyethylene glycol and the like), a preservative, an antioxidant and the like.

When the agent of the present invention is prepared as an aqueous suspension, cells are suspended in the above-described aqueous liquid to obtain a cell density of about 1.0×10⁵ to 1.0×10⁸ cells/ml, preferably about 1.0×10⁶ to 1.0×10⁷ cells/ml.

Because the preparation thus obtained is safe and less toxic, it can be safely administered to mammals (for example, humans, monkeys, dogs, cats, mice, rats, rabbits, sheep, pigs and the like), preferably to humans. Although the subject of administration is preferably the mammalian individual itself from which the active ingredient hematopoietic stem/progenitor cell-like cell is derived, this is not to be construed as limiting, as far as the HLA type of the hematopoietic stem/progenitor cell-like cell administered and the recipient's HLA type match each other. The method of administration is not particularly limited, and the preparation can be administered orally or non-orally, preferably by injection or drip infusion, including intravenous administration, subcutaneous administration, intraperitoneal administration and the like.

The dosage of the cellular immunotherapeutic agent of the present invention varies depending on the activity and choice of the active ingredient, purpose of administration, seriousness of illness, recipient animal species, the recipient's drug receptivity, body weight, age and the like, and cannot be generalized; however, when the agent is used as an alternative treatment to bone marrow (hematopoietic stem cell) transplantation, the dosage is normally 10⁶ cells/kg or more, preferably 10⁶ to 10¹⁰ cells/kg, more preferably 2×10⁶ to 10⁹ cells/kg, based on the amount of active ingredient, per day for an adult.

The cellular immunotherapeutic agent of the present invention can also be administered as an auxiliary therapeutic agent for bone marrow transplantation, along with marrow cells. For example, in case of allogenic bone marrow transplantation, graft-versus-host disease can occur because production of donor-derived blood/immune cells is sometimes delayed, and also because there is not always complete matching of HLA types. In such cases, it is possible to help the transferred cells to take, provided that hematopoietic stem/progenitor cell-like cells are prepared and multiplied from a portion of the donor's marrow cells by the method of the present invention, and thereafter a cell population induced to differentiate into various blood cells to some extent is prepared by a technique known per se, and the cell population is prepared as a preparation and administered by the same means as the above.

The present invention also provides a method of producing blood cells, comprising culturing the aforementioned hematopoietic stem/progenitor cell-like cell under conditions for induction of differentiation into blood cells, and mature blood cells obtained by the method. Mature blood cells include, for example, T cells, B cells, dendritic cells, erythrocytes, macrophages and the like. The medium and other culturing conditions for inducing differentiation into these blood cells are obvious to those skilled in the art. These mature blood cells can include not only hematopoietic stem/progenitor cell-like cells differentiated to have normal function, but also those having a particular function restored by gene therapy, or those having a particular property conferred by a gene modification or special culturing conditions.

Furthermore, the present invention provides a cellular immunotherapeutic agent comprising the above-described mature blood cell. Currently, as materials for preparing mature blood cells, patient-derived monocytes, peripheral circulating hematopoietic stem cells, HLA-matched umbilical blood and the like are assumed, but these stem/progenitor cells are subject to limitations as to the capability of multiplication. According to the present invention, starting hematopoietic stem/progenitor cell-like cells can be supplied without limitations, making it possible to obtain desired amounts of mature blood cells easily.

It is also possible to create various series of mature blood cells from ES cells or iPS cells using the method of the present invention. Preparing stem/progenitor cells having E2A once inactivated therein and then multiplying them for differentiation induction using the method of the present invention is preferred to the method wherein differentiation into a particular type of blood cells is induced after multiplying ES/iPS cells, because it enables much more efficient artificial generation of various blood cells. Examples

The present invention is hereinafter described more specifically by means of the following Examples, which, however, are for illustrative purposes only and do not limit the scope of the invention in any way.

### Example 1: Preparation of mouse hematopoietic stem/progenitor cells by transfer of the Id3 gene

Fig. 1 shows procedures for preparing hematopoietic stem/progenitor cells by transfer of the Id3 gene.

### (1) Culturing conditions for B progenitor cells

B progenitor cells were cultured using a medium containing 10% FCS, 200 U/ml penicillin, 200 ng/ml streptomycin, and 4 mM L-glutamine with the addition of IL7 (10 ng/ml), SCF (10 ng/ml), and FLT3 ligand (10 ng/ml).

### (2) Isolation of hematopoietic stem/progenitor cells from fetal mouse liver

Fetal mouse livers were stained with a mixture of antibodies against differentiation antigens that are specific for various series of blood cells (Lineage markers; Lin). Lin-negative cells were separated using a cell sorter.

### (3) Transfer of the Id3 gene

10⁴ Lin-negative cells separated from a population of fetal liver cells were seeded onto the stroma cell TSt-4 in monolayer culture in a 24-well flat-bottomed plate. A retrovirus incorporating Id3 and a cDNA of TAC antigen (MSCV-Id3-IRES-TAC) was added to the medium to infect the Lin-negative cells therewith. 2 days later, TAC-positive cells were isolated using a cell sorter.

### (4) Preparation of B progenitor cells having Id3 expressed forcibly therein

10⁶ hematopoietic progenitor cells having Id3 expressed forcibly therein (TAC-positive cells) were seeded to a 10cm dish, and co-cultured with the stroma cell S17 in monolayer culture. SCF, IL-7, and Flt3-L (10 ng/ml each) were added to the medium, and the cells were cultured for 14 days. A large number (about 10⁸ cells) of B220-positive CD19-negative pro-B cell-like cells were produced (Fig. 1). Hereinafter, these cells are referred to as IdHP (Id-induced hematopoietic progenitor) cell.

### Example 2: In vitro proliferation potential/differentiation potential analysis of IdHP cells

### (1) In vitro proliferation potential of IdHP cells

If IdHP cells continue to be cultured under the conditions used for initial induction, they continue to proliferate for several months while in a homogeneous state without changing its morphology and surface antigen type. The proliferation rate was at the pace of about twice in 3 days. If it is assumed that cultivation was continued without discarding cells at the time of passage, the cells would have been multiplied more than one million folds during 2 months.

### (2) In vitro differentiation potential analysis of IdHP cells

The differentiation potential of IdHP cells multiplied by being cultured for about 1 month was analyzed using an in vitro differentiation induction system. When myeloid series colony production capability was examined by colony assay, about 30 myeloid series colonies were noted in a plate having 10⁴ IdHP cells seeded thereto. Next, to test the potential for differentiation into T cells, 10³ IdHP cells were seeded onto the stroma cell TSt-4/DLL1 in monolayer culture in a 24-well flat-bottomed plate. 2 weeks later, about 10⁴ CD4/CD8-double-positive cells emerged. Therefore, IdHP cells were thought to retain the potential for differentiation into the myeloid series and T series.

### Example 3: Hematopoietic reconstruction capability of IdHP cells

To demonstrate the possession of the potential as hematopoietic stem cells, it is necessary to demonstrate the possession of the capability of continuing to produce cells of the primary series in vivo for a long period. In case of mice, the determination is made on the basis of whether or not a plurality of series of cells are detectable in peripheral blood for 8 weeks or more after transplantation to mice exposed to a lethal dose of radiation. Hence, 10⁶ IdHP cells were transferred to each mouse exposed to a lethal dose of radiation. In this experiment, 2×10⁵ normal marrow cells, as competitor cells, were transferred at the same time. IdHP cells and normal marrow cells are distinguishable using the surface antigen markers Ly5.1 (IdHP cells) and Ly5.2 (normal marrow cells). 2 weeks later, in the mice receiving the same number of control cells (cells infected with an Id3-free viral vector), no blood cells derived from the transferred cells were seen, but the IdHP cells had produced myeloid series and erythroid series cells (Fig. 2).
2 months later, IdHP cell-derived myeloid series as well as T cells and B cells were detected. If E2A is inhibited, the starting cells should not differentiate into B cells; it is thought that in some of the cells, the expression of Id3 ceased and the activity of E2A was restored.
These results show that the IdHP cells were able to reconstruct hematopoiesis over a long period, that is, have the potential as hematopoietic stem cells.

### Example 4: Salvage of mice from exposure death and maintenance of hematopoietic potential using IdHP cells

To determine whether or not the blood cells created from IdHP cells function normally in vivo, the following experiment was performed. Whether or not mice exposed to a lethal dose of radiation could be salvaged from exposure death was examined. 13 mice exposed to 8 Gy were divided into two groups. A first group of 7 mice did not have the cells transferred thereto. A second group of 6 mice had 10⁷ IdHP cells per mouse transferred thereto after exposure. All of the mice without the transfer died by day 17, whereas the mice with the transfer survived with 4 (67%) being alive 3 weeks later and 2 (33%) being alive even 8 weeks later (Fig. 3A). This result shows that by transferring IdHP cells, exposure death could be avoided at least for a given length of period.
In the peripheral blood of mice surviving for 4 weeks and for 12 weeks, IdHP cell-derived myeloid series as well as T cells and B cells were detected; it was shown that a plurality of series of cells continued to be produced for 3 months or more (Fig. 3B).

### Example 5: Differentiation pluripotency test of individual IdHP cells

When each of IdHP cell was re-seeded to a 96-well plate in the same culturing environment (see Example 1(4)), cell proliferation was seen in more than 50% of the wells. After each clone was multiplied sufficiently, 10⁷ IdHP cells of each of several clones were intravenously injected to radiation-exposed RAG-deficient mice (because the recombination enzyme RAG is lacking, differentiation is inhibited due to the incapability of VDJ recombination, so that mature T cells and B cells are not generated.). 4 weeks later, peripheral blood was collected from each mouse and differentiation marker analysis was performed; as a result, in all clones, reconstruction of multiple series of cells was seen (Fig. 4). These results show that individual IdHP cells are pluripotent progenitor cells, and that IdHP cells can be cloned.

### Example 6: Preparation of hematopoietic stem/progenitor cells using PI polyamide

Synthesis being outsourced to Gentier Biosystems, Inc. (Kyoto City), the synthetic PI polyamide shown in Fig. 5A was obtained. Because this PI polyamide is capable of binding specifically to the E box sequence in genomic DNA, E2A protein is no longer able to bind to the target sequence, and its transcription regulatory function is inhibited (Fig. 5B). Mouse hematopoietic progenitor cells as obtained by the method of Example 1(2) were cultured under the same culturing conditions as in Example 1(4) except that the above-described PI polyamide was added to the medium to obtain a concentration of 10 µM. 10 days later, in the cultivation in the presence of the PI polyamide, B cells decreased remarkably, and a CD19-negative Mac1-negative group of cells increased (Fig. 5C, left), compared with the control (the PI polyamide not added) cultivation. From each culture, the same number of cells were retransferred to a T cell differentiation induction environment. 14 days later, production of a large number of T cells from the cells cultured in the presence of the PI polyamide was seen (Fig. 5C, right). These results show that progenitor cells having the potential for differentiation into T cells multiplied during the cultivation in the presence of the PI polyamide.

### Example 7: Preparation of human hematopoietic stem/progenitor cells by transfer of the Id3 gene and confirmation of pluripotency

A retrovirus incorporating the human Id3 gene was infected to human umbilical blood CD34-positive hematopoietic progenitor cells. 2 days later, the infected cells were separated using a cell sorter, and co-cultured with the stroma cell TSt-4 in the presence of hSCF, hIL-7, and hIL-3 (10 ng/ml each) (Fig. 6A).
When the cells as of 4 weeks after the start of cultivation were examined for FS/SS profile, larger blastoid cells had multiplied selectively in human Id3-expressing cells, compared with a control (cells infected with an Id3-free viral vector) (upper panel in Fig. 6B). According to surface antigen marker analysis, a large number of B cells had emerged in the control, whereas B cells had disappeared and CD33-positive CD19-negative cells had proliferated in the human Id3-expressing cells (human IdHP cells). As the cultivation was continued, the human IdHP cells proliferated continuously like mouse IdHP cells.
When the control cells and human IdHP cells were separately cultured in the presence of IL-2, NK cells (CD56-positive cells) were generated from the human IdHP cells. When the cells were cultured in the presence of GM-CSF, CD11c-positive dendritic cells were generated from the human IdHP cells (Fig. 6C). These results show that the human IdHP cells retain pluripotency.

### Industrial Applicability

Using the method of the present invention, with the only provision that HLA types match each other in bone marrow transplantation, it is possible to prepare cells having characteristics of hematopoietic stem/progenitor cells on the basis of a few cells, and multiply them nearly infinitely, so that the burden on the marrow fluid donor lessens significantly. Furthermore, because it also becomes possible to repeatedly perform transplantation using the multiplied cells, the scope of application of transplantation widens, and even in cases where transplantation is currently unfeasible because of limitations on the number of hematopoietic stem cells, transplantation can become feasible. For example, umbilical blood is often unusable for transplantation because of the small number of hematopoietic stem cells; even in such cases, however, umbilical blood can become usable by producing hematopoietic stem/progenitor cell-like cells by the method of the present invention. More importantly, it also becomes possible to transplant the recipient's own cells as hematopoietic stem cells. Therefore, even in cases where no stem cell donor is found, bone marrow transplantation becomes possible. This means that anticancer agents become usable without fearing bone marrow suppression, not only for diseases for which bone marrow transplantation is a decisive treatment, like leukemia, but also for various other cancers. Because various series of mature blood cells can be created artificially from hematopoietic stem/progenitor cell-like cells obtained by the method of the present invention, the mature blood cells obtained can also be used as cells used for cytotherapy, and cells induced to differentiate to some extent can also be used as auxiliary cells in the event of graft rejection in hematopoietic stem cell transplantation.
While the present invention has been described with emphasis on preferred embodiments, it is obvious to those skilled in the art that the preferred embodiments can be modified. The present invention intends that the present invention can be embodied by methods other than those described in detail in the present specification. Accordingly, the present invention encompasses all modifications encompassed in the gist and scope of the appended "CLAIMS."
This application is based on a patent application No. 2008-061542 (filing date: March 11, 2008) filed in Japan, the contents of which are incorporated in full herein. In addition, the contents disclosed in any publication cited herein, including patents and patent applications, are hereby incorporated in their entireties by reference, to the extent that they have been disclosed herein.

SEQUENCE LISTING

<110> RIKEN
KATSURA, Yoshimoto

<120> METHOD FOR PRODUCING CELLS HAVING CHARACTERISTIC OF HEMATOPOIETIC STEM
CELLS/PROGENITOR CELLS

<130> S2928 EP

<140> EP 09 71 9288.4
<141> 2009-03-11

<150> JP 2008-061542
<151> 2008-03-11

<160> 2

<170> PatentIn version 3.5

<210> 1
<211> 10
<212> DNA
<213> Artificial Sequence

<220>
<223> /note="Description of artificial sequence: E2A binding motif"

<400> 1
aacagatggt 10


<210> 2
<211> 8
<212> DNA
<213> Artificial Sequence

<220>
<223> /note="Description of artificial sequence: E2A binding motif"

<400> 2
gcaggtgk 8

<110>  RIKEN
       KATSURA, Yoshimoto

<120>  Method of Producing Cells Having Characteristics of Hematopoietic
       Stem/Progenitor Cell

<130>  091378

<150>  JP 2008-061542
<151>  2008-03-11

<160>  2

<170>  PatentIn version 3.5

<210>  1
<211>  10
<212>  DNA
<213>  Artificial Sequence

<220>
<223>  E2A binding motif

<400>  1
aacagatggt                                                            10


<210>  2
<211>  8
<212>  DNA
<213>  Artificial Sequence

<220>
<223>  E2A binding motif

<400>  2
gcaggtgk                                                               8

## Claims

1. A method of producing hematopoietic stem/progenitor cell-like cells retaining differentiation pluripotency and self-replication potential, comprising the following steps:
(1) a step for providing a mammalian pro-B cell or progenitor cell thereof, and
(2) a step for culturing the cell according to (1) above under conditions for induction of differentiation into B cells, wherein the function and/or expression of the transcription factor E2A is suppressed at least at the stage of pre-pro-B cells or pro-B cells in the step (2) above.

2. The method according to claim 1, wherein the function of E2A is suppressed using an Id factor.

3. The method according to claim 1, wherein the expression of E2A is suppressed using an antisense nucleic acid, siRNA or ribozyme against the E2A gene.

4. The method according to claim 1, wherein the function of E2A is suppressed by suppressing the function and/or expression of another transcription factor that is under the control of E2A, or that works in cooperation with E2A.

5. The method according to claim 1, wherein the function of E2A is suppressed using a pyrrole-imidazole polyamide.

6. The method according to any one of claims 1 to 5, wherein the mammal is a human.

7. The method according to any one of claims 1 to 6, wherein the pro-B cell progenitor cell is selected from the group consisting of hematopoietic stem cells, hematopoietic progenitor cells, lymphomyeloid series progenitor cells, pre-pro-B progenitor cells, ES cells and iPS cells.

8. A hematopoietic stem/progenitor cell-like cell retaining differentiation pluripotency and self-replication potential, that can be produced by the method according to any one of claims 1 to 7.

9. A cellular immunotherapeutic agent comprising the cell according to claim 8.

10. A method of producing a blood cell, comprising culturing the cell according to claim 8 under conditions for induction of differentiation into blood cells.

11. A mature blood cell that can be produced by the method according to claim 10.

12. A cellular immunotherapeutic agent comprising the cell according to claim 11, or a cell population in the midst of differentiation into the mature blood cell.

13. The agent according to claim 9 or 12, further comprising a marrow cell.
